**Europäisches Patentamt**

(19) **European Patent Office**

**Office européen des brevets**

(11) Publication number: **0 167 690**
**B1**

(12) **EUROPEAN PATENT SPECIFICATION**

(45) Date of publication of patent specification: **30.01.91**

(21) Application number: **84305399.2**

(22) Date of filing: **08.08.84**

(51) Int. Cl.⁵: **A 61 K 9/50**

(54) **Droplet generation.**

(30) Priority: **11.07.84 CA 458605**

(43) Date of publication of application:
**15.01.86 Bulletin 86/03**

(45) Publication of the grant of the patent:
**30.01.91 Bulletin 91/05**

(84) Designated Contracting States:
**AT BE CH DE FR GB IT LI LU NL SE**

(56) References cited:
**GB-A-2 094 833**
**GB-A-2 119 734**
**US-A-4 352 883**
**US-A-4 391 909**

(73) Proprietor: **CONNAUGHT LABORATORIES LIMITED**
**1755 Steeles Avenue West**
**Willowdale Ontario M2N 5T8 (CA)**

(72) Inventor: **Hommel, Martin**
**141 Erskine Avenue**
**Toronto, Ontario, M4P 1Y9 (CA)**
Inventor: **Sun, Anthony Mein-Fang**
**4 Barkwood Crescent**
**Willowdale, Ontario, M2H 3G6 (CA)**
Inventor: **Goosen, Mattheus Florentius Albertus**
**18 Wellington Street**
**Kingston Ontario K7L 3CI (CA)**

(74) Representative: **Burford, Anthony Frederick et al**
**W.H. Beck, Greener & Co. 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is concerned with droplet generation, particularly with respect to droplet generation in the encapsulation of living cells or individual cells in microcapsules.

Various attempts have been made over the past twenty years to provide semi-permeable microcapsules which were both biocompatible with the body tissue and impermeable to the components of the immune system. Typical of such attempts is that of Franklin Lim described in US—A—4,352,883, US—A—4,391,909, GB—A—046029, GB—A—2094833 and GB—A—2119734.

As set forth therein, living tissue or individual cells are suspended in an aqueous solution of a reversibly-gellable material, typically sodium alginate, and droplets of this suspension are allowed to drop into a hardening solution, typically calcium chloride. The temporary capsules so formed are then treated with polylysine and polyethyleneimine to form an outer semi-permeable coating. The core material is reliquified by ionexchange of the calcium ions.

Survival times of microcapsules produced by this prior art procedure in the animal body were consistently less than 3 weeks, thereby severely limiting the utility of this prior art encapsulation procedure in the treatment of diseases requiring organ transplantation, such as diabetes and liver disease.

In our copending European Patent Application Serial No. 83303354.1 filed June 9, 1983, there is described an improvement on the above-mentioned prior art procedure which forms a semi-permeable membrane which is both biocompatible and yet is able to protect transplanted tissue and cells from destruction by the immune system, such that, in animal tests, a single intraperitoneal transplant of encapsulated islets reversed the diabetic state for more than one year.

The success of the procedure according to the aforesaid application results from a semi-permeable and durable membrane which has an outer surface of biocompatible negatively-charged material. The improved durability, i.e. resistance to rupture, of these microcapsules is due to their near perfect spherical shape and enhanced capsule membrane thickness.

Although the microcapsules produced in the aforesaid pending application represent a significant advance in the treatment of diseases requiring organ transplantation, there is one drawback which inhibits more ideal utilization of the microcapsules and this drawback arises from the relatively large size of the individual microcapsules, which have a diameter from 700 to 1000 μm. Microcapsules produced according to the procedure of the Lim patents also had relatively large diameters of about 1000 to 2000 μm. Microcapsules having these diameters cannot be injected directly into the cardiovascular system, since they would occlude the blood vessel. Accordingly, the microcapsules must be implanted into large body cavities, such as the intraperitoneal cavity.

Location of the implants in an area of the body other than the cardiovascular system results in an increase in the response time of the microcapsules to changing blood conditions, since the microcapsules are not directly in contact with the blood stream. In addition, the relatively large size of the microcapsules compared to the microcapsulated tissue or cells (e.g. about 200 μm for islets of Langerhans) results in a high diffusional resistance for molecules passing through the microcapsule core.

An air jet-syringe pump extrusion method was used in the procedure of the aforementioned pending application and in the Lim patents to produce gel droplets containing entrapped islets, or other tissue or cells, from the suspension of the islets in aqueous sodium alginate solution. In this procedure, the sodium alginate solution is extruded through a needle located inside a sheathed tube through which air flows at a controlled rate. As liquid droplets are forced out of the end of the needle by the syringe pump, the droplets are pulled off by the shear forces set up by the rapidly-flowing air stream. The higher the volumetric air flow rate, the stronger are the shear forces, the more quickly the droplets are pulled off the end of the needle and the smaller are the resultant droplets.

However, there are inherent restraints in this prior art procedure which prevent the size of microcapsule produced thereby being less than 700 microns. These restraints are that the viscosity of the gel-forming liquid must be greater than 30 cps (mPa.s) in order to form perfectly spherical capsules, the minimum internal diameter of the needle must be greater than 300 μm (24 gauge) so as to prevent blockage of the needle by the islets, and the volumetric air flow rate must remain below 2000 cm³/min in order to produce capsules of uniform diameter.

We have now discovered an improved procedure and apparatus for forming perfectly spherical, smooth and uniform droplets, such that there can be produced therefrom perfectly spherical, smooth and uniform microcapsules having a diameter of less than 700 μm, preferably about 150 to about 500 μm. Such microcapsules constitute one aspect of the present invention.

The novel microcapsules are formed of biocompatible material and contain living tissue or cells as a core material. A preferred core material is islets of Langerhans, so as to effect long term control of blood sugar levels in diabetic animals, including humans, by cardiovascular injections of biocompatible microencapsulated islets of Langerhans.

The provision of smaller diameter microcapsules in accordance with this invention, for example 200 to 300 μm, permits direct injection of the microcapsules into the blood stream, so that they may eventually lodge inside body organs, such as the liver or spleen, where they are continuously washed with fresh blood. The direct contact between the microcapsule and the bloodsignificantly decreases the response time of the encapsulated tissue or cells to any biochemical change and thereby increases its efficiency. In addition, the smaller microcapsules result in a lower diffusional resistance for molecules passing through the microcapsule core, further increasing the efficiency of the cells. As a result, fewer of the smaller diameter microencapsulated islets of Langerhans need to be transplanted per kilogram of recipient body weight to achieve prolonged control of blood sugar levels in diabetic patients.

The living tissue-containing microcapsules also may be injected or implanted into any other convenient location in the body to be treated thereby, although this manner of administration is less preferred for the reasons noted above.

The small diameter microcapsules provided in accordance with this invention are formed by employing an electrostatic droplet generator in the initial gel-droplet-forming step. In this procedure a droplet, suspended from a source, is charged with a high static voltage and a second location, for example, a collecting vessel, is charged with opposite polarity, so as to attract the droplet. When a threshold of voltage difference between the locations is passed, the droplet moves from the source towards the second location and, thereby, to the collecting vessel.

An adjustable high voltage pulse is generated and applied to the droplet formed on the end of a needle by a syringe pump. The height of the voltage pulse, the pulse frequency and the pulse length are synchronized with the amount of material dispensed, so that known sizes of droplets can be repeatedly generated and collected.

The invention is described further, by way of illustration, with reference to the accompanying drawings in which:

Figure 1 is a schematic representation of a droplet-forming generator constructed in accordance with one embodiment of the invention;

Figure 2 is a schematic representation of a droplet-forming generator, constructed in accordance with a second embodiment of the invention;

Figure 3 is a schematic block diagram representation of the circuitry required to apply electricity to the droplet generator of Figure 1 or 2; and

Figure 4 is a graphical representation of a typical waveform of the output of the droplet generator circuitry of Figure 3.

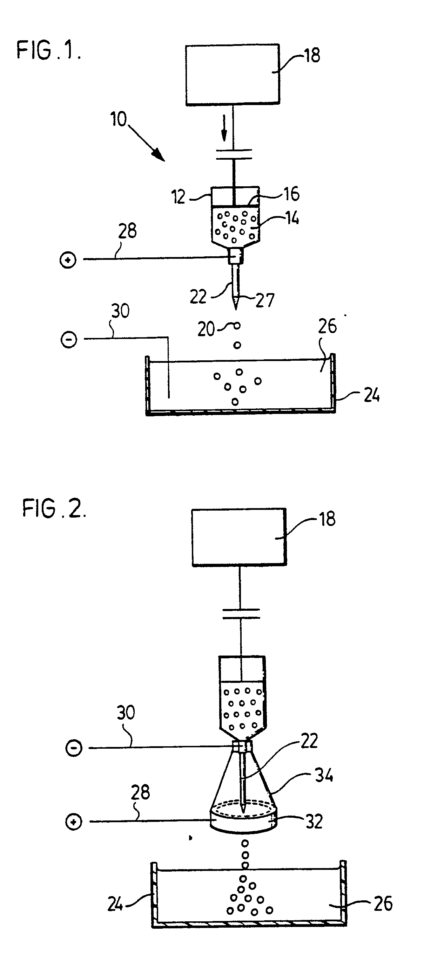

Referring to the drawings, Figure 1 shows a droplet-forming apparatus 10 constructed in accordance with one embodiment of the invention. As shown therein, a syringe 12 of nonconducting, usually polymeric, material contains a gel droplet-forming liquid 14 which contains living cells. A plunger 16 is driven by a syringe pump 18 to expel droplets 20 from the lower end of a stainless steel syringe needle 22 communicating with the lower end of the syringe reservoir 12, towards a collecting vessel 24 containing a hardening solution 26, which may be aqueous calcium chloride solution in the case of an aqueous droplet-forming liquid containing sodium alginate.

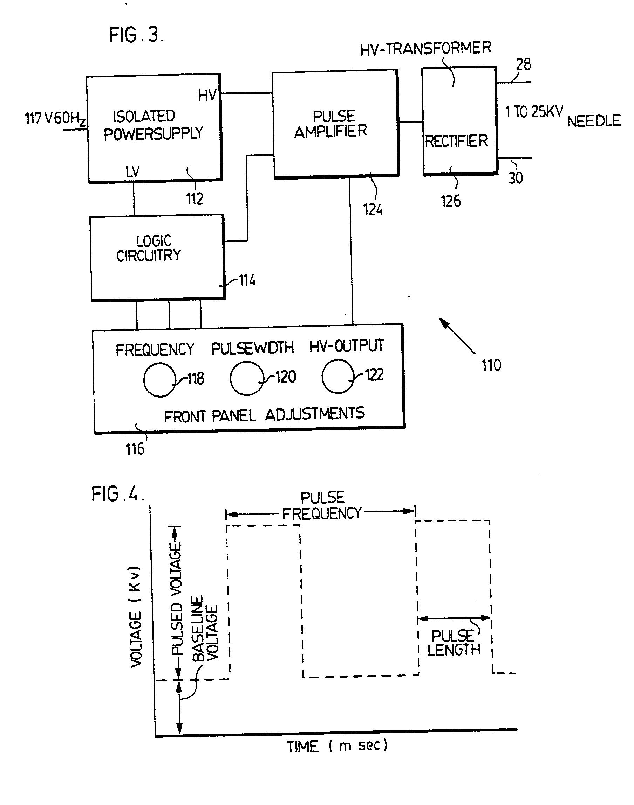

The positive lead 28 of an electrical pulse generator (see Figure 3) is connected to the needle 22 while the negative lead 30 of the pulse generator is connected to the hardening solution.

The needle 22 may be bevelled at its outer tip 27, if desired. The tip 27 is located at a specific distance from the top of the recipient medium 26 in the collecting vessel 24 consistent with the voltage pulse to be applied therebetween to effect droplet formation. The size of the droplets 18 may be varied by varying the distance between the needle tip 27 and the liquid in the collecting vessel 24, with shorter distances leading to smaller droplets, by varying the voltage applied by the leads 28 and 30 with increased voltage leading to smaller droplets, by varying the pulse length of applied electricity with decreasing pulse length leading to smaller droplets, or by varying the speed of the pump 18 with decreasing pump speed leading to smaller droplets.

Figure 2 illustrates an alternative arrangement wherein the positive wire 28 from the pulse generator is detached from the needle 22 and instead is attached to a stainless steel ring 32 which is mounted to the lower end of a conical support 34 of non-conductive material which surrounds and extends below the needle 22. The negative lead 30 is attached to the needle 22 rather than to the recipient medium 26. In this arrangement the distance between the tip 27 of the needle 22 and the top of the recipient medium 26 does not affect the gel droplet size.

The static voltage which is applied by lead wires 28 and 30 during droplet formation in Figures 1 and 2 results in gel droplets having a diameter less than about 700 μm, preferably about 150 to about 500 μm. These droplets then may be coated with a thin coating of a semipermeable biocompatible membrane. The resulting microcapsules are small enough to be injected into an animal body using an 18 gauge needle fitted to a syringe.

Since the voltage applied during droplet formation is a static one, the viability of encapsulated living tissue, such as islets or Langerhans or liver cells, is not destroyed, and hence the microencapsulated living tissue is capable of on-going metabolism.

Referring now to Figures 3 and 4 of the drawings, an electrostatic pulse generator 110 suitable for formation of electrostatic pulses to be applied during droplet formation by the apparatus of Figure 1 or 2 is illustrated in Figure 3. As seen therein, the pulse generator 110 includes an isolated power supply 112, which may be connected to any desired source of electric power, logic circuitry 114, console panel 116 having adjusting knobs for pulse frequency 118, pulse width 120 and high voltage output 122, a pulse amplifier 124, and a high voltage transformer and rectifier 126 which outputs to the electrical lead wires 28 and 30.

The electrical pulse voltage, pulse frequency and pulse length which pass to the droplet forming apparatus 10 by the lead wires 28 and 30 may each vary widely, depending on the size of droplets desired. The pulse voltage, which determines the strength of the force pulling the droplets from the end of the needle 22, usually varies from about 1 to about 25 KV. The pulse frequency, which determines how many pulses are applied to the droplet, usually varies from about 10 to about 100 sec$^{-1}$. The pulse length, which determines the length of time for which the droplet-forming force is applied, usually varies from about 1 to about 6 m. sec. The interaction of the various time periods and their meaning is further illustrated in Figure 4. These values are synchronized with the amount of material dispensed from the needle to obtain uniformly-sized droplets.

These specific design parameters ensure that there is no voltage overlap since a pulse lasts for 1 to 6 m. sec with each pulse occurring every 10 to 100 m. sec. Accordingly, a minimum of 4 m. sec and a maximum of 99 m. sec occurs between pulses. There is, in addition, a low baseline voltage which maintains the forming droplet in position between pulses.

There are numerous examples in the prior art of devices for electrostatically sorting biological cells, electrostatic sprays for dispensing paints and/or polymers and electrostatic droplet generators for ink printing. Illustrative examples of such devices are described in U.S. Patents Nos. 4,347,935, 4,395,716 and 4,097,373 and British Patent No. 1,346,301. The droplet generators described in these prior patents use an external excitation source, such as acoustic vibration, for the initial formation of the droplet. The droplets are charged electrostatically only after they leave the generator, while in the present invention, the droplet-forming liquid 14 is charged directly by a high static voltage. In the prior art arrangements, the external vibrating source causes formation of the droplets while in the present invention droplets are produced by direct electrostatic interaction.

The droplet generator of this invention is capable of producing very small, spherical droplets containing living cells with each step of the droplet formation being under the direct control of the operator.

In one aspect of this invention, living tissue or individual cells are encapsulated in a biocompatible semi-permeable membrane, in the form of a hydrogel. The material to be encapsulated is suspended in a physiologically-compatible medium containing a water soluble substance which can be reversibly gelled to provide a temporary protective environment for the tissue. The medium is formed into droplets containing the tissue, using the droplet generation procedure of the invention, and gelled, for example, by changing conditions of temperature, pH or ionic environment, to form temporary capsules, of substantially perfect spherical shape. Thereafter, the temporary capsules which result are treated to form a membrane of controlled permeability and negatively-charged outer surface about the shape-retaining temporary capsules. The semi-permeable nature of the membrane permits nutrients and oxygen to flow to the core material and metabolic products to flow therefrom while retaining the core material within the microcapsule. The biocompatible nature of the semi-permeable membrane allows the passage of such materials to and from the core to occur without inflammation or other adverse body response while the outer negatively-charged surface inhibits surficial cell growth, so that the membrane remains semi-permeable and effective for extended periods of time, typically from three to six months or longer.

The temporary capsules may be formed from any non-toxic water-soluble substance that can be gelled to form a shape retaining mass by a change of conditions in the medium in which it is placed, and also comprises plural groups that are readily ionized to form anionic or cationic groups. The presence of such groups enables surface layers of the capsule to cross-link to produce a permanent membrane when exposed to polymers containing multiple functionalities of the opposite charge.

Preferably, the temporary capsules are formed from a polysaccharide gum, which may be natural or synthetic, of a type that can be gelled to form a shape retaining mass by exposure to a change in conditions and can be permanently cross-linked or hardened by polymers containing reactive groups, such as amino groups, which can react with the acidic polysaccharide constituents. Most preferably, the gum is alkali metal alginate, specifically sodium alginate, although other water-soluble gums may be used.

The temporary capsules may be formed from sodium alginate by extruding droplets of aqueous sodium alginate solution into an aqueous calcium chloride solution. It is preferred that the temporary capsules be substantially spherical so that perfectly spherical microcapsules can be formed for cardiovascular injection. Substantially perfectly spherical temporary capsules are formed by using an aqueous sodium alginate solution having a viscosity of at least about 30 centipoise (mPa.s). At viscosities below this critical lower limit, the temporary capsules have an irregular shape. Perfectly spherical capsules are obtained over a wide range of viscosity of the sodium alginate solution above the critical lower limit of 30 centipoise (mPa.s) with an upper limit being dictated largely by the ability to extrude the solution into the hardening medium. However, it has also been found that the minimum size of perfectly spherical droplet which can be obtained at a viscosity of at least about 30 cps (mPa.s) increases with increasing viscosity.

Formation of the permanent semi-permeable membrane about the temporary capsules preferably is effected by ionic reaction between free acid groups in the surface layer of the gelled gum and biocompatible polymers containing acid-reactive groups, such as, amino groups, typically in a dilute aqueous solution of the selected polymer.

The cross-linking biocompatible polymers which may be used include polyamino acids, preferably polylysine. It is noted that polyethyleneimine and other imine-containing polymers are unsuitable for membrane formation in view of their non-biocompatible nature. The molecular weight of the preferred polylysine polymer should be controlled within a narrow range of about 10,000 to about 30,000, preferably about 17,000, to achieve the required membrane porosity. The use of polylysine or other polyamino acid results in microcapsules having a positively-charged surface, which would be unsuitable for long term viability, although the microcapsules are biocompatible. It is important for long term in vivo life for the polylysine or other polyamino acid to be reacted for a period of time sufficient to develop a substantial thickness of membrane, so as to provide a substantial number of surface groups for post-reaction, as discussed below, sufficient structural strength to permit in vivo injection and sufficient quantity of biocompatible polymer to permit in vivo structural integrity to be retained. Usually, for polylysine of the molecular weight range noted above, a reaction time of at least six minutes is required to achieve these results, generally up to about 9 minutes. These reaction times result in a polylysine layer thickness of about 5 µm.

Surprisingly, the actual strength of the aqueous solution of polylysine used to react with the temporary capsules does not affect the capsule wall thickness, at concentration levels in excess of about 0.05 wt.%.

The semi-permeable membrane formed about the temporary capsules by the reaction with the polayamino acid next is treated with a non-toxic biocompatible water-soluble polymeric material which is capable of ionic reaction with free amino groups to form an outer negatively-charged coating about the membrane, typically by suspension of the microcapsules in an aqueous solution of the polymeric material. The material used to form the outer coating preferably is the same material as is used to form the temporary capsules, preferably a polysaccharide gum, more preferably an alkali metal alginate, such as, sodium alginate. Other biocompatible polymeric materials containing base-reactive groups, such as, polyvinyl alcohol and poly beta-hydroxy butyric acid, may be used to form the outer coating to the microcapsules. Molecular weights of such polymeric materials typically vary from about $10^4$ to about $10^6$.

The biocompatible water-soluble polymeric material containing amino-reactive groups reacts with the outer amino-groups of the semi-permeable membrane to form an outer coating. This outer coating permanently shrouds the polyamino acid layer, although leaving intact the porosity of the semi-permeable membrane, and provides a negatively-charged surface. By virtue of the number of surface amino groups on the polyamino acid membrane, resulting from the prolonged reaction time, the outer negatively-charged polymer coating resists degradation and removal, in vivo, so that the positively charged surfaces are not exposed to the body environment.

The treatment of the polyamino microcapsules with the biocompatible base-reactive material retains the overall biocompatible nature of the semi-permeable membrane and results in a negatively-charged outer surface which inhibits cell growth and, therefore, permits the semi-permeable membrane to retain its permeability and hence effectiveness over an extended period of time.

Following formation of the microcapsules, reliquification of the suspending medium for the core material may be effected by re-establishing the conditions under which the material is liquid. This may be achieved by ion exchange to remove multivalent cation, for example, by immersion in phosphate buffered saline or citrate buffer. The reliquification step, though beneficial in decreasing diffusion resistance, it is not essential for the provision of an effective product and may be omitted, since it has been shown that transplanted islets (rat to mouse) in microcapsules whose interiors have not been reliquified, are also effective in normalizing blood sugar levels of diabetic animals. Surprisingly, the calcium alginate gel core does not reliquify inside the body, since intact gel cores have been found in microcapsules recovered from diabetic animals up to one year after implantation.

The process of the invention may be used to encapsulate living tissue, multicellular fractions thereof or individual cells, for example, islets of Langerhans, liver cells and red blood cells. The microcapsules which result may be implanted into an appropriate site within a mammalian body for the purpose of providing the body with the specialized physiological function of the tissue while the tissue remains viable. The implantation may be achieved by simple injection, so that surgical procedures are not required. As noted earlier, cardiovascular injection may be effected, in view of the smaller diameter microcapsules which result from the electrostatic droplet generation procedure.

The core of the microcapsules contain the living tissue cells and an aqueous medium of nutrients sufficient to maintain the tissue and allow its normal metabolism. The cells are viable, physiologically active and capable of ongoing metabolism.

The biocompatible semi-permeable membrane encapsulating the core material consists of interpenetrating layers of ionically-interacted biocompatible materials. The overall wall thickness of the semi-permeable membrane usually varies from about 4 to about 6 µm. The microcapsules themselves have a diameter in the range of less than about 700 µm, preferably in the range of about 150 to about 500 µm for microcapsules containing

islets of Langerhans as the core material. The biocompatible semi-permeable membrane is in the form of a hydrogel and hence has an overall water content within the membrane structure of at least about 20 wt%, which may vary up to about 95 wt%, depending on the molecular weight of the polyamino acid.

In a particularly preferred embodiment of the invention, living cells are microencapsulated within a polylysine-alginate semi-permeable hydrogel. The cells are initially suspended uniformly in a sodium alginate solution in physiological saline. Where the microcapsules are to be used for the treatment of diabetes by controlling blood sugar in animals, including humans, the living cells take the form of islets of Langerhans from an animal pancreas.

Spherical droplets containing the cells are produced from an aqueous sodium alginate solution by the electrostatic droplet generation procedure of the invention and are collected as gelled spheres in a hardening solution, such as, calcium chloride. The gelled spheres are coated with polylysine followed by an outer coating of sodium alginate. The microcapsules may then be suspended in isotonic sodium citrate or other convenient ion exchange medium to reliquify the alginate gel inside the microcapsule to restore the cells to a mobile state. As noted earlier, this step may be omitted, if desired.

The outer biochemically inert but biocompatible alginate surface is a negatively-charged hydrogel containing up to about 95% water. The low interfacial tension between the swollen gel surface and the aqueous biological environment minimizes protein interaction, otherwise a strong protein-polymer interaction may cause a severe inflammatory response. The biocompatibility of the hydrogel membrane leads to long term viability of the capsules when implanted. Polyethyleneimine-surface microcapsules do not appear to possess this property, since they produce a strong inflammatory response and hence are rejected by the body, which severely limits the useful in vivo life of the microcapsules. The soft rubbery consistency of most hydrogels may also contribute to their biocompatibility by decreasing frictional irritation to surrounding tissues.

The strength of the microcapsules may be increased by additional cross-linking, for example, using glutaraldehyde, prior to reliquification of the gel, if effected.

For in vivo implantation, it is not essential that the biocompatible outer surface be composed of sodium alginate, but it is essential that the outer surface be biocompatible and negatively-charged. Binding occurs between the negatively-charged groups, usually hydroxyl or carboxyl groups, of the biocompatible outer surface material, and the positively-charged amino groups on polylysine.

By the present invention, therefore, there have been obtaned biocompatible microcapsules capable of long term in vivo life and having a diameter which render them suitable for injection of living tissue into the blood stream, so that the microcapsules may lodge inside body organs for ongoing metabolism therein. While the primary benefit of the smaller diameter microcapsules of the invention is in in-vivo uses, the living tissue-containing microcapsules may also be put to a variety of in-vitro uses.

In addition to producing microcapsules containing living tissue or cells, the present invention may be used to form microcapsules containing a variety of other core materials, depending on the intended end use of the microcapsules.

The invention is illustrated further by the following Examples:

Example 1

This Example illustrates the formation of small diameter gel droplets using an electrostatic droplet generator.

An apparatus as illustrated in Figure 1 was set up. A 1.5% w/v sodium alginate solution (14) was placed in a 10 cm³ syringe (12) to which is attached a 22 gauge stainless steel needle (22) having a 90° bevel outlet. The positive polarity wire (28) was attached to the metal leur lock section of the needle and the needle-syringe combination was attached to the syringe pump (18). A 1.1% calcium chloride solution (26) was poured into a 4" × 1" (10 cm × 2.5 cm) petri dish (24) to which was attached the negative polarity wire (30). The petri dish (24) was positioned so that the liquid surface therein was 10 mm from the tip of the needle (22).

The pulse voltage dial (122) on the adjustment panel (116) was set at 12 KV, the pulse frequency dial (118) at 20 sec$^{-1}$, the pulse length dial (120) at 2 m. sec, and the syringe pump speed at 4 ml/hr. The syringe pump (18) and droplet generator were both turned on so that sodium alginate liquid droplets (20) were drawn from the tip (27) of the needle (22) and, upon entering the calcium chloride solution in the petri dish (24), calcium alginate gel droplets were formed and were collected therein. The resultant calcium alginate gel droplets were found to be perfectly smooth and spherical and with a mean diameter of 300 (±50 SD) μm.

The syringe needle (22) used in this Example was of the same diameter as was previously used in an air jet syringe wherein a rapidly flowing air stream was used to remove sodium alginate liquid droplets from the tip (27) of the needle (22) using the air jet syringe, the smallest diameter calcium alginate gel droplets attainable had a diameter of 700 μm. The electrostatic procedure described in this Example, therefore, was able to decrease the gel droplet diameter to approximately half this value.

Example 2

This Example illustrates the formation of small diameter gel droplets using an alternative form of droplet generation.

The procedure of Example 1 was repeated, except that the apparatus of Figure 2 was utilized, i.e. the negative polarity wire (30) was attached to the needle (22) and the positive polarity wire (28) is attached to the metal ring device (32) which is spaced downwardly from the tip of the needle (22). The centre of the metal ring (32) was positioned 7 mm downwardly from the tip (27) of the needle (22) and an uncharged petri dish (24) was positioned about 5 cm downwardly from the ring assembly.

The calcium alginate gel droplets produced by this procedure and collected in the petri dish were observed to be perfectly smooth and spherical and to have a mean diameter of 450 (±65 SD) μm. When the experiment was repeated with the charge reversed, a greater variation of gel droplet diameter was observed with the standard deviation (SD) of gel droplet diameter approximately doubling.

### Example 3

This Example illustrates the viability of living tissue after passage through the electrostatic droplet generator.

The procedure of Example 1 was repeated except that islets of Langerhans extracted from the pancreatic tissue of dogs were added to the sodium alginate solution in the syringe in a concentration of 500 islets/2 ml and the calcium chloride solution was replaced by saline, so that gel droplet formation did not occur in this experiment. After passage through the electrostatic droplet gegnerator , 100% of the islets were shown to be viable using Trypan blue staining. All the islets appeared white when viewed under the microscope, there being no evidence of the blue appearance characteristic of dead islets.

### Example 4

This Example illustrates the formation of small semi-permeable microcapsules containing islets of Langerhans.

Cultured rat islets of Langerhans (2 × 10$^3$ islets in 0.2 ml medium) were suspended uniformly in 2 ml of a 1.5% (2/2) sodium alginate solution (viscosity 51 cps (mPa.s)) in physiological saline. Spherical droplets containing islets were produced with an electrostatic droplet generator using the procedure of Example 1 and were collected in 1.5% (w/w) calcium chloride solution. The supernatant was decanted and the gelled spherical calcium alginate droplets, containing islets, were washed with CHES (2-cyclohexyl-aminoethane sulfonic acid) solution and 1.1% calcium chloride solution.

After aspirating off the supernatant, the gelled droplets were incubated for 6 minutes in 0.05% (w/w) solution of polylysine having a molecular weight of 17,000. The supernatant was decanted and the polylysine capsules were washed with dilute CHES, 1.1% calcium chloride solution and physiological saline.

The washed polylysine capsules were incubated for 4 minutes in 30 ml of 0.03% sodium alginate membrane on the initial polylysine membrane, by ionic interaction between the negatively-charged alginate and the positively-charged polylysine.

The resulting microcapsules were washed with saline, 0.05M citrate buffer for 6 months to reliquify the inner calcium alginate, and a final saline wash. The microcapsules were found to be perfectly spherical and each to contain from 1 to 2 viable islets. The microcapsules had a mean diameter of 300 (±50 SD) μm and wall thicknesses of 5 μm. The microcapsules were suspended in nutrient medium at 37°C.

The viability of the islets was demonstrated by Trypan Blue staining after the capsule walls were dissociated with heparin.

### Example 5

This Example illustrates the formation of small semi-permeable microcapsules containing hepatocytes (liver cells).

The procedure of Example 4 was repeated except that fetal mouse or adult rat hepatocytes were added to the sodium alginate solution in amounts of 10$^5$ hepatocytes/ml of alginate solution and the distance from the tip of the needle to the surface of the calcium chloride solution was decreased to 7 mm. The resulting microcapsules were spherical in appearance and had a diameter of 250 μm (±50 SD). The presence of viable hepatocytes was demonstrated by Trypan Blue staining and histology, even after more than 4 weeks in culture at 37°C.

### Example 6

This Example illustrates the effect of needle parameters of gel droplet size.

The procedure of Example 1 was repeated, except that a 26 gauge needle having a 22-degree bevel was used in place of the 22 gauge needle having the 90-degree bevel. The resultant gel droplets had a diameter of 170 μm (±30 SD), demonstrating the smaller diameter gel droplets and consequently microcapsules can be formed by using a smaller diameter needle.

In summary of this disclosure, the present invention provides a novel droplet generation procedure using electrostatic forces which is particularly useful in the microencapsulation of living tissue or cells to form small diameter microcapsules suitable for cardiovascular injection. Modifications are possible within the scope of the invention.

**Claims**

1. Spherical, smooth and uniform microcapsules having

a core comprising one or more viable, healthy, physiologically-active tissue or cells capable of on-going metabolism and an aqueous medium of nutrients sufficient to maintain the tissue cells and allow normal metabolism thereof, and

a biocompatible semi-permeable membrane comprised of ionically-interacted materials surrounding and enclosing the core and being permeable to tissue nutrients and metabolic products produced by the tissue but impermeable to immune system proteins,

characterized in that said microcapsules have a diameter of less than 700 micrometres and have a negatively-charged outer surface, whereby they are suitable for cardiovascular injection into an animal body.

2. Microcapsules as claimed in Claim 1 having a diameter of 150 to 500 micrometres.

3. Microcapsules as claimed in Claim 1 or Claim 2, wherein the membrane has a thickness of 1 to 6 micrometres.

4. Microcapsules as claimed in any one of the preceding claims, wherein the tissue cells are islets of Langerhans.

5. Microcapsules as claimed in any one of the preceding claims, wherein said tissue or cells are entrapped within a reliquifiable reversible gel material.

6. Microcapsules as claimed in Claim 5, wherein said reversible gel material is calcium alginate.

7. Microcapsules as claimed in any of Claims 1 to 4, wherein said tissue or cells are entrapped within reliquified material.

8. Microcapsules as claimed in Claim 7, wherein said reliquified gel material is aqueous sodium alginate.

9. Microcapsules as claimed in any one of Claims 3 to 8, wherein said semi-permeable membrane is formed about each of the microcapsules by ionic reaction between free acid groups in the surface layer of the gelled reversible gel material and a biocompatible polymer containing acid-reactive groups.

10. Microcapsules as claimed in Claim 9, wherein said biocompatible polymer is a polyamino acid.

11. Microcapsules as claimed in Claim 10 wherein the polyamino acid is a polylysine having a molecular weight of 10,000 to 30,000.

12. Microcapsules as claimed in Claim 10 or Claim 11, wherein said negatively-charged surface is provided by reacting the biocompatible polymer, after reaction with the gelled reversible gel material, with a non-toxic biocompatible water-soluble polymeric material capable of ionic reaction with free amino groups.

13. Microcapsules as claimed in Claim 12, wherein said polymeric material is a polysaccharide gum.

14. Microcapsules as claimed in Claim 13, wherein said polysaccharide gum is an alkali metal alginate.

15. A method of forming spherical, smooth and uniform microcapsules containing living tissue or cells and having a diameter of less than 700 micrometres, said method comprising suspending said tissue or cells in an aqueous solution of a reversibly-gellable material, forming droplets of said suspension and allowing said droplets to drop into a hardening solution, characterized in that said drops are formed by extruding said suspension downwardly from a source at a first location, charging the extruded material with a charge of one polarity, establishing a charge of opposite polarity at a second location spaced vertically below the first location, and providing a difference in voltage between the first and second locations sufficient to form a liquid droplet from the extruded material and draw the same towards the second location.

16. A method as claimed in Claim 15, wherein said first location comprises an axially-downwardly directed electroconductive needle, and said second location comprises said hardening solution.

17. A method as claimed in Claim 15, wherein said first location comprises an axially-downwardly directed electroconductive needle, said second location comprises a ring of electroconductive material surrounding the axis of the needle and being concentric with the axis and said hardening solution is located below the second location.

18. A method as claimed in any one of Claims 15 to 17, wherein the voltage difference for droplet formation is applied cyclically in pulses to continuously-extruded suspension to effect continuous production of droplets.

19. A method as claimed in Claim 18, wherein the pulse voltage is 1 to 25 KV, the pulse frequency is 10 to 100 sec$^{-1}$, and the pulse duration is 1 to 6 m. sec.

20. A method as claimed in any one of Claims 15 to 19, wherein the materials and/or conditions used are such as to provide microcapsules as claimed in any one of Claims 1 to 14.

**Patentansprüche**

1. Sphärische, glatte und gleichförmige Mikrokapseln mit einem Kern, der ein oder mehrere lebensfähige, gesunde, physiologisch aktive Gewebstücke oder Zellen aufweist, welche in der Lage sind, einen weiterdauernden Stoffwechsel durchzuführen und ein wässriges Medium mit Nährstoffen, die hinreichend sind, das Gewebe bzw. die Zellen zu versorgen und deren normalen Stoffwechsel zu ermöglichen, und mit

einer biokompatiblen semi-permeablen Membran, welche ionisch wechselwirkende Bestandteil aufweist, die den Kern umgeben und einschließen sowie permeabel sind für Gewebe-Nährstoffe und Stoffwechselprodukte, welche durch das Gewebe erzeugt werden, jedoch nicht permeabel sind für Immunsystem-Proteine, dadurch gekennzeichnet daß die genannten Mikrokapseln einen Durchmesser von weniger als 700 Mikrometer und eine negativ geladene äußere Oberfläche aufweisen, wodurch sie geeignet sind für eine kardiovaskuläre Injektion in einen Tierkörper.

2. Mikrokapseln gemäß Anspruch 1, mit einem Durchmesser von 150 bis 500 Mikrometer.

3. Mikrokapseln gemäß einem der Ansprüche 1 oder 2, wobei die Membran eine Stärke von 1 bis 6 Mikrometer aufweist.

4. Mikrokapseln gemäß einem der vorhergehenden Ansprüche, wobei die Gewebe-Zellen sogenannte Langerhans-Inseln sind.

5. Mikrokapseln gemäß einem der vorhergehenden Ansprüche, wobei genannten Gewebstücke oder Zellen eingeschlossen sind in ein wiederverflüssigbares, reversibles Gel-Material.

6. Mikrokapseln gemäß Anspruch 5, wobei das reversible Gel-Material Calcium-Alginat ist.

7. Mikrokapseln gemäß einem der Ansprüche 1 bis 4, wobei die genannten Gewebstücke oder Zellen eingeschlossen sind in einem wiederverflüssigen Material.

8. Mikrokapseln gemäß Anspruch 7, wobei das genannte wiederverflüssigte Gel-Material ein wässriges Natrium-Alginat ist.

9. Mikrokapseln gemäß einem der Ansprüche 3 bis 8, wobei die genannte semi-permeable Membran um jede der Mikrokapseln gebildet ist durch eine ionische Reaktion zwischen freien Säuregruppen in der Oberflächenschicht des gelierten reversiblen Gel-Materials und einem biokompatiblen Polymer, welches säurereaktive Gruppen enthält.

10. Mikrokapseln gemäß Anspruch 9, wobei das genannte biokompatible Polymer eine Polyaminosäure ist.

11. Mikrokapseln gemäß Anspruch 10, wobei die Polyaminosäure ein Polylysin ist mit einem Molekulargewicht von 10 000 bis 30 000.

12. Mikrokapseln gemäß einem der Ansprüche 10 oder 11, wobei die genannte negativ geladene Oberfläche derart gebildet wird, daß das biokompatible Polymer, nach einer Reaktion mit dem gelierten reversiblen Gel-Material, mit einem nicht-toxischen biokompatiblen, wasserlöslichen Polymer-Material zur Reaktion gebracht wird, welches in der Lage ist, eine Ionenreaktion mit freien Aminogruppen durchzuführen.

13. Mikrokapseln gemäß Anspruch 12, wobei das genannte polymere Material ein Polysaccharid-Gummi ist.

14. Mikrokapseln gemäß eine der Ansprüche 13, wobei als polysaccharides Gummi ein Alkalimetall-Alginat vorgesehen ist.

15. Verfahren zum Erzeugen sphärischer, glatter und gleichförmiger Mikrokapseln, die lebendes Gewebe oder Zellen enthalten undeinen Durchmesser von weniger als 700 Mikrometer haben, wobei das Verfahrern folgende Schritte aufweist: Eintauchen des genannten Gewebes oder der Zellen in eine wässrige Lösung aus reversibel gellierbarem Material, Formen von Tropfen der gennanten Suspension und Einbringen der genannten Tropfen in eine Härter-Lösung, dadurch gekennzeichnet, daß die genannten Tropfen derart gebildet werden, daß die Suspension aus einer Quelle an einem ersten Ort gezogen wird, daß das gezogene Material mit einer Ladung einer gegebenen Polarität geladen wird, daß eine Ladung umgekehrten Vorzeichens an einem zweiten Ort erzeugt wird, der vertikal unter dem ersten Ort unter Abstand angeordnet ist, und daß eine Spannungsdifferenz zwischen den ersten und zweiten Orten erzeugt wird, die ausreicht, um von dem gezogenen Material einen flüssigen Tropfen zu formen und den selben zum zweiten Ort zu ziehen.

16. Verfahren nach Anspruch 15, wobei der genannte erste Ort eine axial nach unter gerichtete, elektrisch leitende Nadel aufweist und der zweite Ort eine Härter-Lösung.

17. Verfahren nach Anspruch 15, wobei der genannte erste Ort eine axial nach unten gerichtete, elektrische leitende Nadel aufweist und der genannte zweite Ort einen Ring aus elektrisch leitendem Material, der die Achse der Nadel umgibt und konzentrisch ausgerichtet ist in bezug auf die Achse, und daß die genannte Härter-Lösung unterhalb des zweiten Ortes angeordnet ist.

18. Verfahren nach einem der Ansprüche 15 bis 17, wobei die Spannungsdifferenz für die Tropfenbildung zyklisch in Pulsen an die kontinuierlich gezogene Suspension angelegt wird, um eine kontinuierliche Produktion von Tropfen zu erreichen.

19. Verfahren nach Anspruch 18, wobei die Pulsspannung 1 bis 25 KV beträgt und die Pulsfrequenz im Bereich von 10 bis 100 $sek^{-1}$ liegt, während die Pulsdauer im Bereich von 1 bis 6 msek liegt.

20. Verfahren nach einem der Ansprüche 15 bis 19, wobei die Materialien und/oder Bedingungen so gewählt sind, daß Mikrokapseln gemäß einem der Ansprüche 1 bis 14 gebildet werden.

**Revendications**

1. Microcapsules sphériques, lisses et uniformes, possédant un noyau comportant un ou plusieurs tissus ou cellules physiologiquement actifs, sains, viables capables de métabolisme permanent et un milieu aqueux de nutriments suffisant pour conserver les cellules tissulaires et leur permettre un métabolisme normal, et une membrane semi-perméable biocompatible composée de matériaux à interaction ionique entourant et enfermant le noyau et perméable aux nutriments tissulaires et aux produits métaboliques produits par le tissu mais imperméable aux protéines du système immunitaire, caractérisées en ce que lesdits microcapsules possèdent un diamètre inférieur à 700 μm et ont une surface externe chargée négativement, ce qui les rend propres à l'injection cardiovasculaire dans un organisme animal.

2. Microcapsules selon la revendication 1, possédant un diamètre de 150 à 500 μm.

3. Microcapsules selon la revendication 1 ou la revendication 2, dans lesquelles la membrane a une épaisseur de 1 à 6 μm.

4. Microcapsules selon l'une quelconque des revendications précédentes, dans lesquelles les cellules tissulaires sont des ilôts de Langerhans.

5. Microcapsules selon l'une quelconque des revendications précédentes, dans lesquelles ledit tissu ou lesdites cellules sont noyés dans un gel réversible reliquéfiable.

6. Microcapsules selon la revendication 5, dans lesquelles ledit gel réversible est de l'alginate de calcium.

7. Microcapsules selon l'une quelconque des revendications 1 à 4, dans lesquelles ledit tissu ou lesdites cellules sont noyés dans du matériau reliqéfié.

8. Microcapsules selon la revendication 7, dans lesquelles ledit gel reliquéfié est de l'alginate de sodium aqueux.

9. Microcapsules selon l'une quelconque des revendications 3 à 8, dans lesquelles ladite membrane semi-perméable est formée autour de chacune des microcapsules par réaction ionique entre des groupements acide libres dans la couche superficielle du gel reversible gélifié et un polymère biocompatible contenant des groupement réagissant avec les acides.

10. Microcapsules selon la revendication 9, dans lesquelles ledit polymère biocompatible est un acide polyaminé.

11. Microcapsules selon la revendication 10, dans lesquelles l'acide polyaminé est un polylysine de poids moléculaire compris entre 10 000 et 30 000.

12. Microcapsules selon la revendication 10 ou la revendication 11, dans lesquelles ladite surface chargée négativement est obtenue en faisant réagir le polymère biocompatible, après réaction avec le gel réversible gélifiée, avec un matériau polymère hydrosoluble biocompatible non toxique capable de réaction ionique avec les groupements aminés libres.

13. Microcapsules selon la revendication 12, dans lesquelles ledit matériau polymère est une gomme polysaccharidique.

14. Microcapsules selon la revendication 13, dans lesquelles ladite gomme polysaccharidique est un alginate de métal alcalin.

15. Procédé de formation de microcapsules sphériques, lisses et uniformes contenant du tissu ou des cellules vivants et ayant un diamètre inférieur à 700 µm, ledit procédé comprenant la mise en suspension dudit tissu ou desdites cellules dans une solution aqueuse d'un matériau réversiblement gélifiable, la formation de gouttelettes à partir de ladite suspension et le dégoutte- ment desdites gouttelettes dans une solution de durcissement, caractérisé en ce que lesdites gouttes sont formées en extrudant ladite suspension vers le bas à partir d'une source en un premier site, en chargeant le matériau extrudé avec une charge d'une polarité, en établissant une charge de polarité opposée en un second site placé à distance verticalement en dessous du premier site et en appliquant une différence de tension entre le premier et le second site suffisante pour former une gouttelette de liquide à partir du matériau extrudé et attirer cette gouttelette vers le second site.

16. Procédé selon la revendication 15, dans lequel ledit premier site comprend une aiguille électroconductrice dirigée axialement vers le bas et ledit second site comprend ladite solution de durcissement.

17. Procédé selon la revendication 15, dans lequel ledit premier site comprend une aiguille électroconductrice dirigée axialement vers le bas, ledit second site comprend un anneau de matériau électroconducteur entourant l'axe de l'aiguille, concentrique par rapport à l'axe, et ladite solution de durcissement est située en dessous du second site.

18. Procédé selon l'une quelconque des revendications 15 à 17, dans lequel la différence de tension destinée à la formation des gouttelettes est appliquée cycliquement par impulsion à la suspension extrudée continûment, pour obtenir une production continue de gouttelettes.

19. Procédé selon la revendication 18, dans lequel la tension des impulsions est comprise entre 1 à 25 kV, la fréquence des impulsions entre 10 et 100 s$^{-1}$ et la durée des impulsions entre 1 et 6 ms.

20. Procédé selon l'une quelconque des revendications 15 à 19, dans lequel les matériaux et/ou les conditions utilisés sont tels qu'ils fournissent des microcapsules selon l'une quelconque des revendications 1 à 14.

EP 0 167 690 B1

FIG.1.

FIG.2.

FIG. 3.

ISOLATED POWERSUPPLY

117 V 60Hz

HV

LV

112

HV-TRANSFORMER

PULSE AMPLIFIER

124

RECTIFIER

126

28

1 TO 25KV NEEDLE

30

LOGIC CIRCUITRY

114

110

FREQUENCY    PULSEWIDTH    HV-OUTPUT

118            120            122

FRONT PANEL ADJUSTMENTS

116

FIG. 4.

PULSE FREQUENCY

VOLTAGE (Kv)

PULSED VOLTAGE

BASELINE VOLTAGE

PULSE LENGTH

TIME (m sec)